# EUROPEAN PATENT SPECIFICATION

(11) **EP 2 854 236 B1**
(45) Date of publication and mention of the grant of the patent: **21.02.2018**
(21) Application number: 14183921.7
(22) Date of filing: 08.09.2014
(51) Int. Cl.: H01R 13/62, H01R 13/64, H01R 24/84

(54) **System for electrical coupling in a medical device**
System zur elektrischen Kopplung in einer medizinischen Vorrichtung
Système pour le couplage électrique dans un dispositif médical

(30) Priority: 30.09.2013 US 201314041200
(43) Date of publication of application: 01.04.2015
(73) Proprietor: Covidien LP, Mansfield, MA 02048 (US)
(72) Inventor: Pham, Thomas, San Jose, CA 95136 (US)
(74) Representative: Gray, James

(56) References cited:
- WO-A1-2011/024091
- US-A- 5 498 230

## Description

### Technical Field

The present embodiments relate generally to a coupling, and more specifically to systems and methods for coupling first and second connectors to achieve an electrical coupling in a medical device.

### Background

In various medical procedures, some medical equipment is used within a sterile field while other equipment is kept out of the sterile field. However, often the equipment within the sterile field must be operatively connected to the equipment outside the sterile field. For example, in electrosurgical procedures, the treatment device within the sterile field must be connected to an electrosurgical generator, which is typically located outside the sterile field, by a cable that spans the non-sterile and sterile fields.

For example, one electrosurgical procedure that typically uses a treatment device within a sterile field and a generator outside the sterile field is an electrosurgical treatment for venous reflux disease. Various electrosurgical endovenous treatments are commonly used for treating venous reflux disease, and other diseases of hollow anatomical structures (HAS), such as endothermal ablation, Radio Frequency Ablation (RFA), or laser ablation. Venous reflux disease is a disease caused by damaged vein valves, which typically prevent blood from flowing backwards in a vein.

RFA and endothermal ablation typically uses radio frequency heating to create targeted tissue ablation to seal off damaged veins. RFA equipment typically includes an RF generator and a catheter having a heating segment located at the distal end, which is inserted into the vein(s) during treatment. The heating segment uses RF energy driven by the RF generator to heat and seal the vein.

To reduce the spread of disease, the catheter is typically a single-use device, or in some cases may be reused after sterilization. The catheter typically connects to a power cable that supplies power from the RF generator to the heating segment at the distal end of the catheter. The power cable is typically used for multiple procedures by attaching to the catheter without sterilization between procedures, i.e. the power cable is typically outside the sterile field in the operating room, while the catheter is within the sterile field. Thus, procedures for connecting the catheter to the power cable present challenges in maintaining sterility within the sterile field.

WO 2011/024091 (D1) discloses a magnetic connection system suitable for use with a wireless ultrasound probe which utilizes a plurality of magnets to facilitate coupling between said probe and a diagnostic or clinical device in a manner which minimizes the effects of stray magnetic fields on the device.

US 5,498,230 (D2) discloses a sterile connector and video camera including a connector assembly for connecting a sterile endoscope to an unsterile surgical camera and a light fiber cable.

### SUMMARY

The present invention provides a system for electrically coupling two pieces of an electrosurgical system, one piece within the sterile field and one outside the sterile field, that one person within the sterile field can couple without contaminating the sterile field. The various embodiments of the present systems and methods for electrical coupling have several features. Without limiting the scope of the present embodiments as expressed by the claims that follow, their features now will be discussed briefly. After considering this discussion, and particularly after reading the section entitled "Detailed Description," one will understand how the features of the present embodiments provide the advantages described herein.

In general, in one aspect, the implementation of the disclosure features a system for electrical coupling including a treatment apparatus having a proximal end and a distal end. The system also includes an elongate power cable having a proximal end configured for coupling to a power source, and a distal end. A first coupler portion is disposed at the distal end of the power cable, and includes at least a first electrical contact and at least one first magnetic portion. A second coupler portion is disposed at the proximal end of the treatment apparatus, and includes at least a second electrical contact and at least one second magnetic portion, wherein the first and second magnetic portions are magnetically attractable to each other. The magnetic attraction between the first and second magnetic portions draws the first and second coupler portions to one another and causes the first and second coupler portions to releasably couple to one another when the first and second coupler portions are brought into close proximity to one another, thereby coupling the distal end of the power cable to the proximal end of the treatment apparatus. The system further includes a first aligner located on the first coupler portion and a second aligner located on the second coupler portion, wherein the first and second aligners mate to maintain alignment between the first coupler portion and the second coupler portion when the first and second coupler portions couple to one another. The first aligner and the second aligner are selected from the group comprising a protrusion and a recess. The system further includes a sterile sleeve having a proximal end and a distal end, such the distal end of the sterile sleeve is secured to at least one of the treatment apparatus and the second coupler portion. The sleeve is configured to cover at least a portion of the first and second coupler portions when the first and second coupler portions are coupled to one another.

One or more of the following features may be included. The first magnetic portion and the second magnetic portion may be selected from the group comprising a magnet and a ferromagnetic material. The at least a first magnetic portion may be selected from a group comprising a pair of first magnets, a pair of first ferromagnetic materials, and a combination of a first magnet and a first ferromagnetic material.

The pair of first magnets, first ferromagnetic materials, or magnet and ferromagnetic material, may be located adjacent diagonally opposite corners of a coupling area of the first coupler portion, and a mating pair of second magnets, second ferromagnetic material, or second magnet and second ferromagnetic material, may be located adjacent diagonally opposite corners of a coupling area of the second coupler portion.

The sterile sleeve may include a retracted state in which the sterile sleeve covers only portions of at least one of the treatment apparatus and the second coupler portion, and an extended state in which the sterile sleeve covers the first and second coupler portions and at least portions of the power cable.

In general, in another aspect, the implementation of the disclosure features a system for electrical coupling including a treatment apparatus having a proximal end and a distal end. The system also includes an elongate power cable having a proximal end configured for coupling to a power source, and a distal end. A first coupler portion is disposed at the distal end of the power cable, and includes at least a first electrical contact and at least one first magnetic portion. A second coupler portion is disposed at the proximal end of the treatment apparatus, and includes at least a second electrical contact and at least one second magnetic portion, wherein the first and second magnetic portions are magnetically attractable to each other. The magnetic attraction between the first and second magnetic portions draws the first and second coupler portions to one another, causes the first and second coupler portions to self-align with one another, and causes the first electrical contact to make electrical connection with the second electrical contact, thereby coupling the distal end of the power cable to the proximal end of the treatment apparatus. The system may also include a sterile sleeve secured to at least one of the treatment apparatus and the second coupler portion and configured to cover at least a portion of the first and second coupler portions.

One or more of the following features may be included. The first magnetic portion and the second magnetic portion may be selected from the group comprising a magnet and a ferromagnetic material.

The system may include a first aligner located on the first coupler portion and a second aligner located on the second coupler portion, wherein the first and second aligner mate to maintain alignment between the first coupler portion and the second coupler portion. The first aligner and the second aligner may be selected from the group comprising a protrusion and a recess.

The at least a first magnetic portion may be selected from a group comprising a pair of first magnets, a pair of first ferromagnetic materials, and a combination of a first magnet and a first ferromagnetic material. The pair of first magnets, first ferromagnetic materials, or magnet and ferromagnetic material, may be located adjacent diagonally opposite corners of a coupling area of the first coupler portion and a mating pair of second magnets, second ferromagnetic material, or second magnet and second ferromagnetic material, may be located adjacent diagonally opposite corners of a coupling area of the second coupler portion.

The sterile sleeve may include a retracted state in which the sterile sleeve covers only portions of at least one of the treatment apparatus and the second coupler portion, and an extended state in which the sterile sleeve covers the first and second coupler portions and at least portions of the power cable.

In general, in still another aspect, the implementation of the disclosure features a system for electrical coupling including a treatment apparatus having a proximal end and a distal end. The system also includes an elongate power cable having a proximal end configured for coupling to a power source, and a distal end. A first coupler portion is disposed at the distal end of the power cable, and has at least a first electrical contact and at least one first magnetic portion. A second coupler portion is disposed at the proximal end of the treatment apparatus, and has at least a second electrical contact and at least one second magnetic portion, wherein the first and second magnetic portions are magnetically attractable to each other. The magnetic attraction between the first and second magnetic portions draws the first and second coupler portions to one another and causes the first and second coupler portions to releasably couple and self align to one another when the first and second coupler portions are brought into close proximity to one another, such that the first electrical contact makes electrical connection with the second electrical contact, thereby coupling the distal end of the power cable to the proximal end of the treatment apparatus.

One or more of the following features may be included. The first magnetic portion and the second magnetic portion may be selected from the group comprising a magnet and a ferromagnetic material.

The system may further include a first aligner located on the first coupler portion and a second aligner located on the second coupler portion, wherein the first and second aligner mate to maintain alignment between the first coupler portion and the second coupler portion. The first aligner and the second aligner may be selected from the group comprising a protrusion and a recess.

The system may also include a sterile sleeve having a proximal end and a distal end, and configured to cover at least a portion of the first and second coupler portions when the first and second coupler portions are coupled to one another. In certain embodiments, the distal end of the sterile sleeve may be secured to at least one of the treatment apparatus and the second coupler portion. Further, the sterile sleeve may comprise a retracted state in which the sterile sleeve covers only portions of at least one of the treatment apparatus and the second coupler portion, and an extended state in which the sterile sleeve covers the first and second coupler portions and at least portions of the power cable. In various embodiments, the sterile sleeve may also include a rigid or semi-rigid portion and a flexible portion.

The at least a first magnetic portion may be selected from a group comprising a pair of first magnets, a pair of first ferromagnetic materials, and a combination of a first magnet and a first ferromagnetic material. The pair of first magnets, first ferromagnetic materials, or magnet and ferromagnetic material, may be located adjacent diagonally opposite corners of a coupling area of the first coupler portion, and a mating pair of second magnets, second ferromagnetic material, or second magnet and second ferromagnetic material, may be located adjacent diagonally opposite corners of a coupling area of the second coupler portion.

In general, in another aspect, the implementation of the disclosure features a system for electrical coupling including a treatment apparatus having a proximal end and a distal end. The system also includes an elongate power cable having a proximal end configured for coupling to a power source, and a distal end. A first coupler portion is disposed at the distal end of the power cable, and has at least a first electrical contact. A second coupler portion is disposed at the proximal end of the treatment apparatus, and has at least a second electrical contact. The system further includes means for generating an attraction force between the first and second coupler portions. The attraction force between the first and second coupler portions draws the first and second coupler portions to one another and causes the first and second coupler portions to releasably couple to one another when the first and second coupler portions are brought into close proximity without having to touch both coupler portions, thereby coupling the distal end of the power cable to the proximal end of the treatment apparatus. A first aligner may be located on the first coupler portion and a second aligner may be located on the second coupler portion and are configured to maintain alignment between the first coupler portion and the second coupler portion. The system further includes a sterile sleeve configured to cover at least a portion of the first and second coupler portions when the first and second coupler portions are coupled to one another.

One or more of the following features may be included. The means for generating an attraction force may be chosen from the group comprising a magnet and a magnet, and a magnet and a ferromagnetic material.

The first aligner and the second aligner may be selected from the group comprising a protrusion and a recess.

A distal end of the sterile sleeve may be secured to at least one of the treatment apparatus and the second coupler portion. The sterile sleeve may include a retracted state in which the sterile sleeve covers only portions of at least one of the treatment apparatus and the second coupler portion, and an extended state in which the sterile sleeve covers the first and second coupler portions and at least portions of the power cable. The sleeve may include a rigid or semi-rigid portion and a flexible portion.

In general, in still yet another aspect, the implementation of the disclosure features a system for electrical coupling including a treatment apparatus having a proximal end and a distal end. The system also includes an elongate power cable having a proximal end configured for coupling to a power source, and a distal end. A first coupler portion is disposed at the distal end of the power cable, and has at least a first electrical contact. A second coupler portion is disposed at the proximal end of the treatment apparatus, and has at least a second electrical contact. The system further includes means for generating an attraction force between the first and second coupler portions. The attraction force between the first and second coupler portions draws the first and second coupler portions to one another, aligns the first and second electrical contacts, and causes the first and second coupler portions to releasably couple to one another when the first and second coupler portions are brought into close proximity without having to touch both coupler portions, thereby coupling the distal end of the power cable to the proximal end of the treatment apparatus. The system also includes a sterile sleeve configured to cover at least a portion of the first and second coupler portions when the first and second coupler portions are coupled to one another.

One or more of the following features may be included. The means for generating an attraction force may be chosen from the group comprising a magnet and a magnet, and a magnet and a ferromagnetic material.

The system may further comprise a first aligner located on the first coupler portion and a second aligner located on the second coupler portion configured to maintain alignment between the first coupler portion and the second coupler portion. The first aligner and the second aligner may be selected from the group comprising a protrusion and a recess.

A distal end of the sterile sleeve may be secured to at least one of the treatment apparatus and the second coupler portion. The sterile sleeve may include a retracted state in which the sterile sleeve covers only portions of at least one of the treatment apparatus and the second coupler portion, and an extended state in which the sterile sleeve covers the first and second coupler portions and at least portions of the power cable. The sleeve may also include a rigid or semi-rigid portion and a flexible portion.

In general, in another aspect, the implementation of the disclosure features a system for electrical coupling including a treatment apparatus having a proximal end and a distal end. The system further includes an elongate power cable having a proximal end configured for coupling to a power source, and a distal end. A first coupler portion is disposed at the distal end of the power cable, and has at least a first electrical contact. A second coupler portion is disposed at the proximal end of the treatment apparatus, and has at least a second electrical contact. The system also includes means for generating an attraction force between the first and second coupler portions. The attraction force between the first and second coupler portions draws the first and second coupler portions to one another and causes the first and second coupler portions to releasably couple to one another when the first and second coupler portions are brought into close proximity without having to touch both coupler portions, and the first and second coupler portions are configured to self-align when brought into close proximity such that the first electrical contact makes electrical connection with the second electrical contact, thereby coupling the distal end of the power cable to the proximal end of the treatment apparatus.

One or more of the following features may be included. The means for generating an attraction force may be chosen from the group comprising a magnet and a magnet, and a magnet and a ferromagnetic material.

The system may further include a first aligner located on the first coupler portion and a second aligner located on the second coupler portion, wherein the first and second aligners mate to maintain alignment between the first coupler portion and the second coupler portion. The first aligner and the second aligner may be selected from the group comprising a protrusion and a recess.

The system may further include a sterile sleeve having a proximal end and a distal end, the sterile sleeve being configured to cover at least a portion of the first and second coupler portions when the first and second coupler portions are coupled to one another. In embodiments, a distal end of the sterile sleeve may be secured to at least one of the treatment apparatus and the second coupler portion. The sterile sleeve may include a retracted state in which the sterile sleeve covers only portions of at least one of the treatment apparatus and the second coupler portion, and an extended state in which the sterile sleeve covers the first and second coupler portions and at least portions of the power cable. The sterile sleeve may also include a rigid or semi-rigid portion and a flexible portion.

In general, in still another aspect, the implementation of the disclosure features a method of coupling a treatment apparatus to an elongate power cable, wherein the power cable has a first coupler portion with at least a first electrical contact at a distal end of the power cable, and the treatment apparatus has a second coupler portion with at least a second electrical contact at a proximal end of the treatment apparatus. The method includes moving the first and second coupler portions into close proximity with each other, thereby generating an attraction force between the first and second coupler portions, the attraction force causing the first and second coupler portions to releasably couple to one another without the first coupler portion or the power cable contacting an operator. Thereafter, the operator covers the first and second couplers with a sterile sleeve, wherein a distal end of the sterile sleeve is secured to at least one of the treatment apparatus and the second coupler, and wherein covering the first and second couplers comprises moving the sterile sleeve from a retracted state in which the sterile sleeve covers only portions of at least one of the treatment apparatus and the second coupler portion, to an extended state in which the sterile sleeve covers the first and second coupler portions and at least portions of the power cable.

One or more of the following features may be included. The treatment apparatus may be sterile and maintained in a sterile field and the power cable is adjacent to the sterile field. The attraction force may be a magnetic force.

The first and second coupler portions may further include, respectively, first and second mating aligners that maintain alignment between the first and second coupler portions. The first and second aligners may be selected from the group comprising a protrusion and a recess.

The sterile sleeve may also include a rigid or semi-rigid portion and a flexible portion.

In general, in another aspect, the implementation of the disclosure features a method of coupling a treatment apparatus to an elongate power cable, wherein the power cable has a first coupler portion with at least a first electrical contact at a distal end of the power cable, and the treatment apparatus has a second coupler portion with at least a second electrical contact at a proximal end of the treatment apparatus. The method includes moving the first and second coupler portions into close proximity with each other, thereby generating a magnetic attraction force between the first and second coupler portions, the magnetic attraction force causing the first and second coupler portions to releasably couple to one another and thereby causing the first electrical contact to make electrical connection with the second electrical contact without having to touch both coupler portions. The first and second coupler portions further include, respectively, first and second mating aligners that maintain alignment between the first and second coupler portions. Thereafter, the operator covers the first and second couplers with a sterile sleeve, wherein a distal end of the sterile sleeve is secured to at least one of the treatment apparatus and the second coupler portion.

One or more of the following features may be included. The treatment apparatus may be sterile and maintained in a sterile field and the power cable is adjacent to the sterile field.

Covering the first and second couplers may includes moving the sterile sleeve from a retracted state in which the sterile sleeve covers only portions of at least one of the treatment apparatus and the second coupler portion, to an extended state in which the sterile sleeve covers the first and second coupler portions and at least portions of the power cable.

The attraction force may be a magnetic force.

The sterile sleeve may also include a rigid or semi-rigid portion and a flexible portion.

In general, in yet another aspect, the implementation of the disclosure features a method of coupling a treatment apparatus to an elongate power cable, wherein the power cable has a first coupler portion with at least a first electrical contact at a distal end of the power cable, and the treatment apparatus has a second coupler portion with at least a second electrical contact at a proximal end of the treatment apparatus. The method comprises moving the first and second coupler portions into close proximity with each other, thereby generating an attraction force between the first and second coupler portions and causing the first and second coupler portions to releasably couple to one another without having to touch both coupler portions. The first and second coupler portions are configured to self-align when brought into close proximity such that the first electrical contact makes electrical connection with the second electrical contact.

One or more of the following features may be included. The attraction force may be a magnetic force.

The first and second coupler portions may also include, respectively, first and second mating aligners that maintain alignment between the first and second coupler portions. The first and second aligners may be selected from the group comprising a protrusion and a recess.

The method may further include covering the first and second couplers with a sterile sleeve. In embodiments, the sterile sleeve may include a rigid or semi-rigid portion and a flexible portion.

Covering the first and second couplers may include moving the sterile sleeve from a retracted state in which the sterile sleeve covers only portions of at least one of the treatment apparatus and the second coupler portion, to an extended state in which the sterile sleeve covers the first and second coupler portions and at least portions of the power cable.

The treatment apparatus may be sterile and maintained in a sterile field while the power cable is adjacent to the sterile field.

The invention may be implemented to realize one or more of the following advantages. The first and second coupler portions may be connected without having to touch the coupler that is outside the sterile field or is non-sterile, thereby preventing contamination of the operator and the sterile field. The sterile sleeve provides protection to the sterile field by covering the non-sterile components. The magnetic attraction creates automatic alignment between the first and second coupler portions thereby eliminating the need for the operator to touch both coupler portions. The magnetic attraction keeps the first and second coupler portions connected during use, and the first and second aligners provide further support to keep the first and second coupler portions aligned and engaged during use.

### BRIEF DESCRIPTION OF THE DRAWINGS

The various embodiments of the present systems and methods for electrical coupling now will be discussed in detail with an emphasis on highlighting the advantageous features. These embodiments are for illustrative purposes only. These drawings include the following figures, in which like numerals indicate like parts:
Figure 1 is an overview of a medical treatment system;
Figures 2 and 3 are side elevation views of an example procedure using the medical treatment system of Figure 1;
Figure 4 is a side perspective view of a catheter connector and a power cable connector prior to connection;
Figure 5 is a top plan view of the power cable connector of Figure 4;
Figure 6 is a bottom plan view of the catheter connector of Figure 4;
Figure 7 is a side perspective view of the catheter connector and the power cable connector after connection;
Figure 8 is a top plan view of the catheter connector and the power cable connector after connection;
Figure 9 is a top plan view of the catheter connector and the power cable connector after connection and with a protective sleeve extended over a junction of the catheter connector and the power cable connector; and
Figure 10 is a top plan view of the catheter connector and the power cable connector after connection and with the protective sleeve of Figure 9 extended over the junction of the catheter connector and the power cable connector and over a length of the power cable.

The present embodiments provide systems and methods for preventing contamination of a sterile field during a treatment procedure. In particular, the present embodiments include a treatment apparatus that is configured to occupy the sterile field, and a power cable that is configured to remain outside the sterile field. The treatment apparatus is connectable to the power cable via an attraction, such as a magnetic attraction, between the treatment apparatus and the power cable, so that an operator within the sterile field need not touch the power cable in order to couple the treatment apparatus to the power cable. Instead, the operator need only grasp the sterile treatment apparatus and bring it in close proximity to the power cable. The attraction then causes the treatment apparatus to couple with the power cable. A sterile sleeve may then be slid over a junction of the treatment apparatus and the power cable to provide a barrier between the operator and the non-sterile power cable located within the sleeve. In this manner, the treatment apparatus can be coupled to the power cable by a single operator without contaminating the sterile field.

### DETAILED DESCRIPTION

The following detailed description describes the present embodiments with reference to the drawings. In the drawings, reference numbers label elements of the present embodiments. These reference numbers are reproduced below in connection with the discussion of the corresponding drawing features.

Directional terms used herein, such as proximal, distal, upper, lower, clockwise, counterclockwise, etc., are used with reference to the configurations shown in the figures. For example, a component that is described as rotating clockwise when viewed from the perspectives shown in the figures may be said to rotate counterclockwise when viewed from the opposite perspective. Furthermore, the present embodiments may be modified by altering or reversing the positions or directions of movement of various components. Accordingly, directional terms used herein should not be interpreted as limiting.

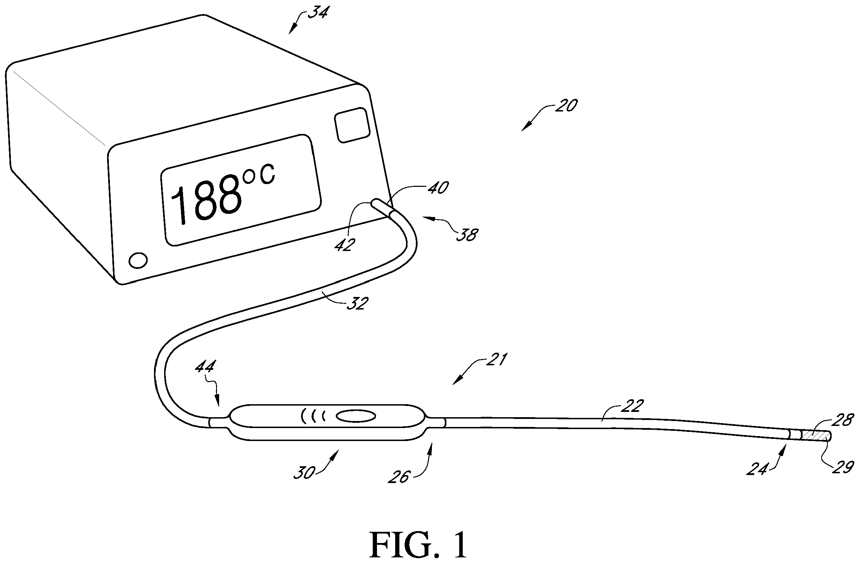

Referring to Figure 1, an exemplary medical treatment system 20 may include a treatment apparatus 21 comprising a catheter shaft 22 having a distal end 24 and a proximal end 26. A heating segment 28 may be operably attached adjacent the distal end 24 of the catheter shaft 22 and a handle 30 is attached at the proximal end 26 of the catheter shaft 22. A power cable 32 electrically connects the heating segment 28 to a power source 34. The power cable 32 may be removably connected to the handle 30 and removably connected to the power source 34. Alternatively, the power cable 32 may be integrally connected to the power source 30. The handle 30 may comprise a first coupler portion and a second coupler portion, where the first and second coupler portions are attached to and/or integral with the power cable 32 and the treatment apparatus 21, respectively.

The heating segment 28 may include a heating element 29. The heating element 29 may in some embodiments be a resistive coil, which may be driven, for example, by RF energy, ultrasound, or any other electrical form. The heating segment 28 is secured at the distal end 24 of the elongate catheter shaft 22. The catheter shaft 22 may be used to maneuver the heating segment 28 into a desired placement within a HAS. In certain embodiments, the catheter shaft 22 is sized to fit within a patient's vascular structure. The proximal end 26 of the catheter shaft 22 includes a handle 30 that may include a connection for interfacing with the power source 34 through the power cable 32, and/or a port for fluid or guidewire passage.

In certain embodiments, the power source 34 comprises an alternating current (AC) source, such as an RF generator. In other embodiments, the power source 34 comprises a direct current (DC) power supply, such as, for example, a battery, a capacitor, or other power source such as would be used for microwave heating. The power source 34 may also incorporate a controller that, through the use of a processor, applies power based at least upon readings from a temperature sensor or sensors (e.g., a thermocouple, a thermistor, a resistance temperature device, an optical or infrared sensor, combinations of the same or the like) located in or adjacent to the heating segment 28. For example, the controller may heat the tissue of a HAS or the heating segment 28 to a set temperature. In an alternative embodiment, the operator selects a constant power output of the power source 34. For example, the operator may manually adjust the power output relative to a temperature display from a temperature sensor in the heating segment 28.

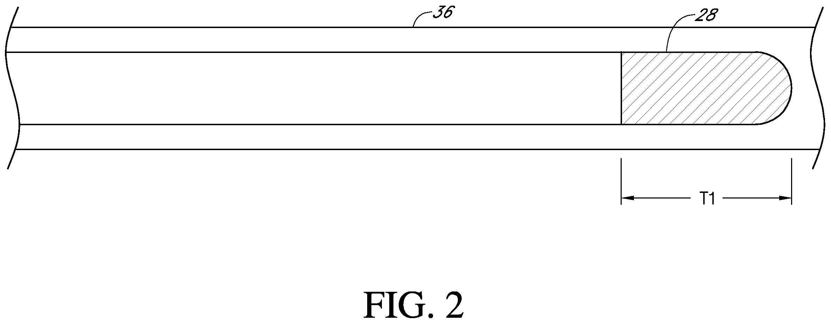

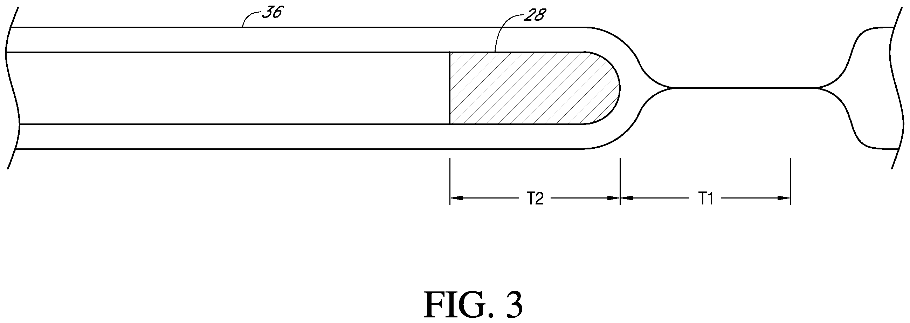

The exemplary medical treatment system 20 may be used in various medical procedures, including endovenous treatments to treat venous reflux. Specifically, referring to Figure 2, a method may comprise inserting the heating segment 28 into a distal-most section of a HAS 36 to be treated. The heating segment 28 is then aligned with a first treatment location T1 within the HAS 36. Power is then applied to the heating segment 28 for a desired length of time to treat the first treatment location T1. After a desired dwell time, such as after the HAS 36 has collapsed as shown in Figure 3, the power supply to the heating segment 28 may be reduced or shut off. With the power off (or substantially reduced), the heating segment 28 may then be moved proximally until the distal end of the heating segment 28 is adjacent to the proximal end of the first treatment location T1, as shown in Figure 3. At this second treatment location T2 within the HAS 36, power is again applied to the heating segment 28 for a desired length of time to treat the HAS 36 at the second treatment location T2. This process is repeated until the treatment of the HAS 36 is complete. In some embodiments, T1 and T2 may overlap.

To reduce the spread of disease, the catheter 22 is typically a single-use device that is provided in a sterile condition at the beginning of a treatment procedure, and disposed of (or re-sterilized) at the end of the treatment procedure. The power cable 32, by contrast, may be used for multiple procedures without sterilization between procedures. The catheter 22 is thus within a sterile field of the procedure, while the power cable 32 is outside the sterile field. Procedures for connecting the catheter 22 to the power cable 32 present challenges in preventing the power cable 32 from contaminating the sterile field, and/or contaminating a operator who is in the sterile field.

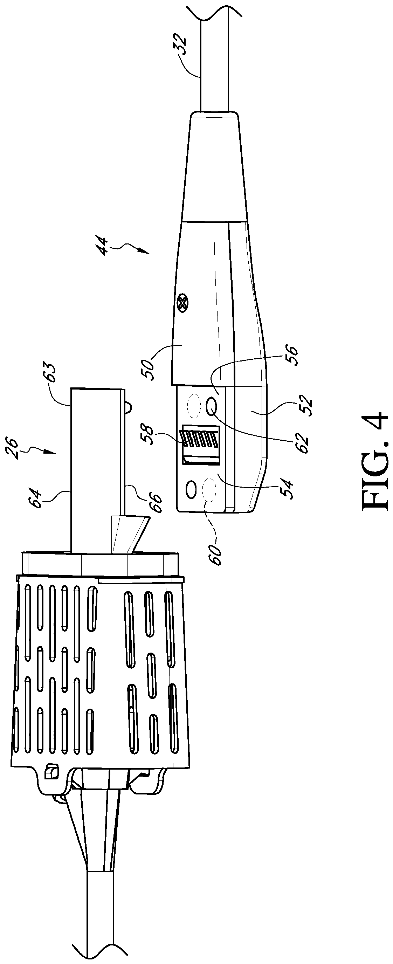

Figures 1 and 4-10 illustrate one embodiment for addressing the foregoing problem. The system of Figures 1 and 4-10 electrically couples a treatment apparatus with a power cable, such as the treatment apparatus 21 and the power cable 32 of Figure 1, or other treatment apparatus (not shown). With reference to Figure 1, the power cable 32 includes a proximal end 38 configured for coupling to the power source 34. In the illustrated embodiment, the proximal end 38 comprises a plug 40 that is received in a port 42 of the power source 34. The power cable further includes a distal end 44, which is configured for coupling to the handle 30 at the proximal end 26 of the treatment apparatus 21. Figures 4-10 illustrate in detail one embodiment of a coupling between the treatment apparatus 21 and the power cable 32.

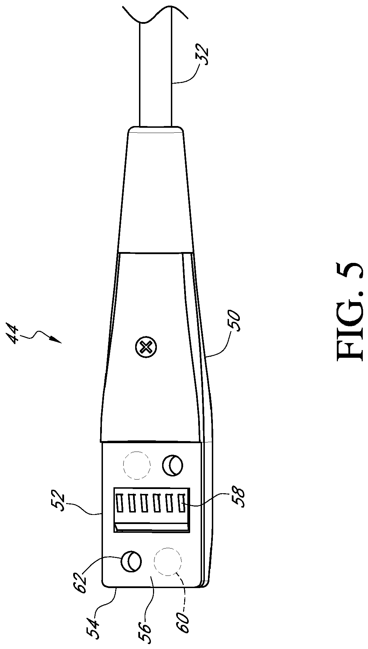

With reference to Figures 4 and 5, the distal end 44 of the power cable 32 includes a first coupler portion 50. The first coupler portion 50 comprises a first body portion 52 defining a first coupler area 54 having a first coupler surface 56, and having at least one first electrical contact 58 and at least one first magnetic portion 60 in the first coupler area 54. In the illustrated embodiment, six first electrical contacts 58 and two first magnetic portions 60 are shown, but in alternative embodiments any number of first electrical contacts 58 and/or first magnetic portions 60 could be provided. The first magnetic portions 60 are illustrated as being internal to the first body portion 52, but in alternative embodiments could be external to the first body portion 52 and/or fitted within recesses in the first body portion 52.

The first coupler portion 50 further includes at least one first aligner comprising a recess 62. In the illustrated embodiment, two recesses 62 are shown, but in alternative embodiments any number of recesses 62 could be provided. Also as illustrated, the first magnetic portions 60 are positioned opposite one another along a first diagonal of the first coupler area 54 and on opposite sides of the first electrical contacts 58, and the recesses 62 are positioned opposite one another along a second diagonal of the first coupler area 54 and on opposite sides of the first electrical contacts 58. However, the illustrated configuration of the first electrical contacts 58, the first magnetic portions 60, and the recesses 62, is merely one example.

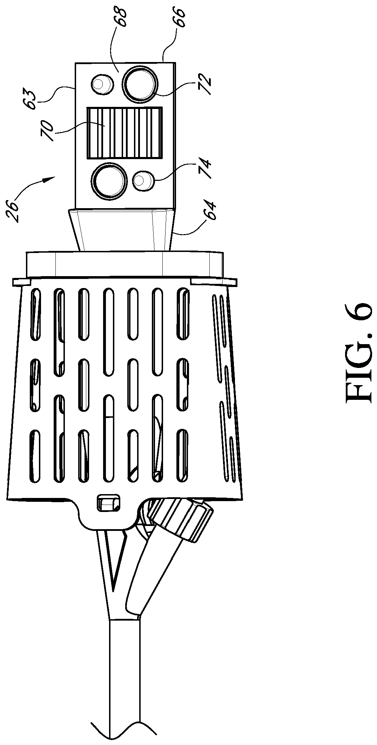

With reference to Figures 4 and 6, the proximal end 26 of the treatment apparatus 21 (Figure 1) includes a second coupler portion 63. The second coupler portion 63 comprises a second body portion 64 defining a second coupler area 66 having a second coupler surface 68 (Figure 6), and having at least one second electrical contact 70 and at least one second magnetic portion 72 in the second coupler area 66. In the illustrated embodiment, six second electrical contacts 70 and two second magnetic portions 72 are shown, but in alternative embodiments any number of second electrical contacts 70 and/or second magnetic portions 72 could be provided. As further described below, the first and second magnetic portions 60, 72 are magnetically attractable to each other and facilitate coupling between the first and second coupler portions 50, 63. The second magnetic portions 72 are illustrated as being external to the second body portion 64, but in alternative embodiments could be internal to the second body portion 64.

With continued reference to Figure 6, the second coupler portion 63 further includes at least one second aligner comprising a protrusion 74. In the illustrated embodiment, two protrusions 74 are shown, but in alternative embodiments any number of protrusions 74 could be provided. Also as illustrated, the second magnetic portions 72 are positioned opposite one another along a first diagonal of the second coupler area 66 and on opposite sides of the second electrical contacts 70, and the protrusions 74 are positioned opposite one another along a second diagonal of the second coupler area 66 and on opposite sides of the second electrical contacts 70. However, the illustrated configuration of the second electrical contacts 70, the second magnetic portions 72, and the protrusions 74, is merely one example.

Each of the first and second magnetic portions 60, 72 may comprise magnets and/or ferromagnetic materials. For example, the first magnetic portions 60 may comprise a pair of first magnets, a pair of first ferromagnetic materials, or a combination of a first magnet and a first ferromagnetic material, and the second magnetic portions 72 may comprise a pair of second magnets, a pair of second ferromagnetic materials, or a combination of a second magnet and a second ferromagnetic material.

In alternative embodiments, the relative positions of the protrusions 74 and the recesses 62 may be reversed. That is, the protrusions 74 may be provided on the first coupler portion 50 and the recesses 62 may be provided on the second coupler portion 63.

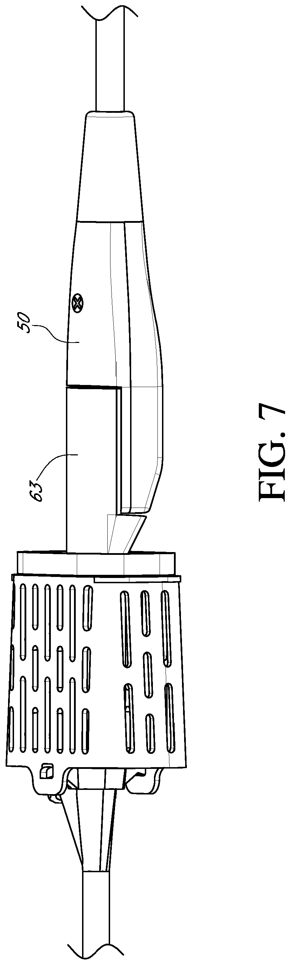

With reference to Figures 4-6, the first magnetic portions 60 are configured to align with the second magnetic portions 72, and the protrusions 74 are configured to align with the recesses 62, when the first and second coupler portions 50, 63 are positioned with the first and second coupler surfaces 56, 68 facing one another, as shown in Figure 4. When the first and second coupler portions 50, 63 are positioned as in Figure 4, and moved in close proximity to one another, a magnetic attraction between the first and second magnetic portions 60, 72 draws the first and second coupler portions 50, 63 to one another, causing the protrusions 74 to seat within the recesses 62, and causing the first and second coupler portions 50, 63 to releasably couple to one another, thereby coupling the distal end 44 of the power cable 32 to the proximal end 26 of the treatment apparatus 21, with the first and second electrical contacts 58, 70 aligning and abutting one another for electrical communication between the power source 34 and the treatment apparatus 21 via the power cable 32 (Figure 1). The mating protrusions 74 and recesses 62 not only facilitate proper alignment between the first and second coupler portions 50, 63, as shown in Figure 7, but also resist separation of the first and second coupler portions 50, 63 from one another under the influence of twisting forces in the plane of the interface between the first and second coupler surfaces 56, 68.

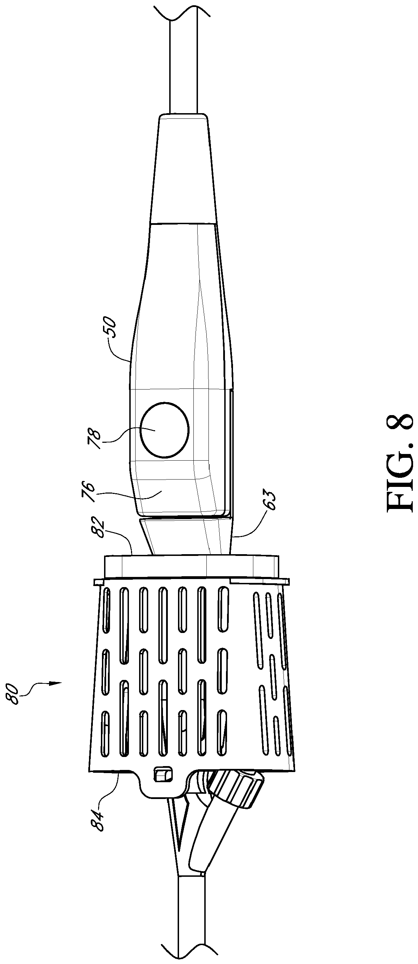

With reference to Figure 8, an upper surface 76 of the first coupler portion 50 may include an on/off switch 78. In the illustrated embodiment, the on/off switch 78 is a button, but in alternative embodiments, the on/off switch 78 could comprise other structures, such as a sliding switch. In some embodiments, depressing and releasing the button initiates power delivery from the power source 34 to the heating segment 28 via the power cable 32 (Figure 1), and depressing and releasing the button a second time ceases power delivery from the power source 34 to the heating segment 28. In other embodiments, depressing and holding the button initiates power delivery from the power source 34 to the heating segment 28 via the power cable 32 (Figure 1), and releasing the button ceases power delivery from the power source 34 to the heating segment 28.

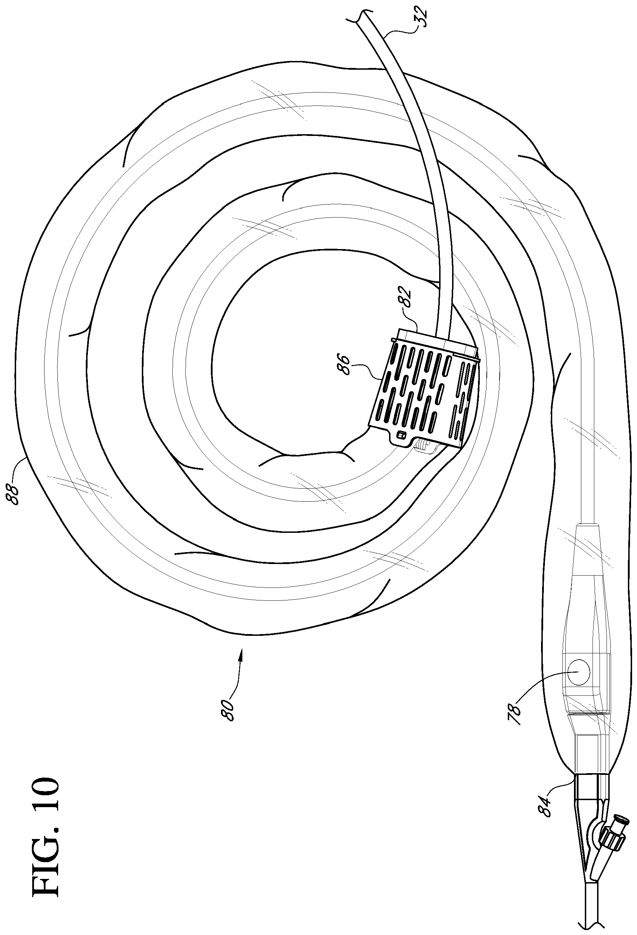

With reference to Figures 8-10, the medical treatment system 20 further comprises a sterile sleeve 80 having a proximal end 82 and a distal end 84. With reference to Figure 10, the distal end 84 of the sterile sleeve 80 is secured to the second coupler portion 63, while the proximal end 82 is unsecured to anything. In alternative embodiments, the distal end 84 of the sterile sleeve 80 could be secured to the treatment apparatus 21 instead of, or in addition to, the second coupler portion 63.

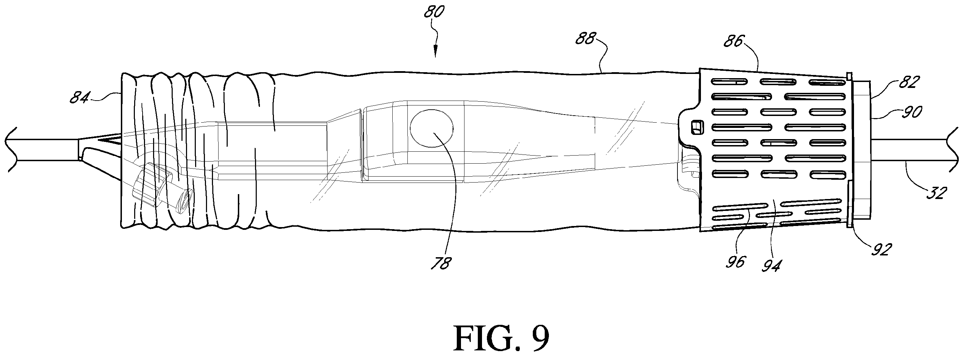

With reference to Figure 8, the sterile sleeve 80 is tubular, and comprises a retracted state in which the sterile sleeve 80 covers only portions of at least one of the treatment apparatus 21 and the second coupler portion 63. With reference to Figures 9 and 10, the sterile sleeve 80 further comprises an extended state in which the sterile sleeve 80 covers the first and second coupler portions 50, 63 and at least portions of the power cable 32.

With reference to Figure 9, the sterile sleeve 80 may comprise a rigid or semi-rigid portion 86 at its proximal end 82, and a flexible portion 88 extending distally from the rigid or semi-rigid portion 86. The flexible portion 88 may comprise a transparent or translucent material that is flexible and durable, and that allows the on/off switch 78 to be visible to the operator and to be manipulated by the operator. For example, the flexible portion 88 may comprise a polymer, such as polyethylene, polypropylene, or any other suitable thermoplastic.

The rigid or semi-rigid portion 86 also comprises a material that is durable, but that also has sufficient rigidity to act as a grip for the operator. While grasping the rigid or semi-rigid portion 86, the operator can pull the flexible portion 88 in the proximal direction until it covers the junction of the first and second coupler portions 50, 63 (Figure 9) and at least a portion of the power cable 32 (Figure 10). For example, the rigid or semi-rigid portion 86 may comprise a polymer, such as nylon, polyethylene, polypropylene, polyurethane, acrylonitrile butadiene styrene (ABS), polyether ether ketone (PEEK), polytetrafluoroethylene (PTFE), thermoplastic polyetherimide (ULTEM), or any other suitable thermoplastic.

In the illustrated embodiment, the rigid or semi-rigid portion 86 is shaped as a short tube having a generally rectangular cross-section with rounded corners, but other cross-sectional shapes may be utilized. An inner diameter of the rigid or semi-rigid portion 86 is sufficient to accommodate the first and second coupler portions 50, 63 with a clearance between the first and second coupler portions 50, 63 and the inner diameter of the rigid or semi-rigid portion 86. The inner diameter of the rigid or semi-rigid portion 86 may also be large enough to accommodate the flexible portion 88 of the sterile sleeve 80 until the sterile sleeve 80 is deployed. In this manner, the rigid or semi-rigid portion 86 provides protection to the flexible portion 88, as well as maintains the flexible portion 88 out of the way in a stored, retracted configuration. With reference to Figure 9, near its proximal end 90, the rigid or semi-rigid portion 86 includes an outwardly extending flange 92 around its periphery. The flange 92 provides a bearing surface for the operator's hand to reduce the likelihood of the operator's hand slipping off the rigid or semi-rigid portion 86 and touching the non-sterile power cable 32 when the operator pulls the sterile sleeve 80 from the retracted state of Figure 8 to the extended state of Figure 10. Distally of the flange 92, a wall 94 of the rigid or semi-rigid portion 86 may include a plurality of spaced openings 96 to provide a better gripping surface for the rigid or semi-rigid portion 86. In alternative embodiments, the wall 94 may be solid.

As described herein, the present embodiments provide systems and methods for preventing contamination of a sterile field during a treatment procedure. In particular, the present embodiments include a treatment apparatus that is configured to occupy the sterile field, and a power cable that is configured to remain outside the sterile field. The treatment apparatus is connectable to the power cable via an attraction, such as a magnetic attraction, between the treatment apparatus and the power cable, so that an operator within the sterile field need not touch the power cable in order to couple the treatment apparatus to the power cable. Instead, the operator need only grasp the sterile treatment apparatus and bring it in close proximity to the power cable. The attraction then causes the treatment apparatus to couple with the power cable. A sterile sleeve may then be slid over a junction of the treatment apparatus and the power cable to provide a barrier between the operator and the non-sterile power cable located within the sleeve. In this manner, the treatment apparatus can be coupled to the power cable by a single operator within the sterile field without contaminating the sterile field.

The foregoing description is intended to illustrate and not limit the scope of the present invention, which is defined by the scope of the appended claims. Other embodiments are within the scope of the following claims. For example, the treatment apparatus 21, while described as a catheter system for the treatment of HAS, may be any treatment apparatus that attaches to a power cable and generator outside of the sterile field. For example, the treatment apparatus can be electrosurgical arthroscopic devices, electrosurgical scalpels, electrosurgical forceps, etc.

## Claims

1. A system (20) for electrical coupling, comprising:
a treatment apparatus (21) having a proximal end (26) and a distal end (24);
an elongate power cable (32) having a proximal end (38) configured for coupling to a power source (34), and a distal end (44);
a first coupler portion (50) at the distal end (44) of the power cable (32), the first coupler portion (50) having at least a first electrical contact (58) and at least one first magnetic portion (60);
a second coupler portion (63) at the proximal end (26) of the treatment apparatus (21), the second coupler portion (63) having at least a second electrical contact (70) and at least one second magnetic portion (72), wherein the first and second magnetic portions (60, 72) are magnetically attractable to each other; and
a sleeve (80) having a proximal end (82) and a distal end (84), the sleeve (80) being configured to cover at least a portion of the first and second coupler portions (50, 63) when the first and second coupler portions (50, 63) are coupled to one another, wherein the sleeve (80) comprises a retracted state in which the sleeve (80) covers only portions of at least one of the treatment apparatus (21) and the second coupler portion (63), and an extended state in which the sleeve (80) covers the first and second coupler portions (50, 63) and at least a portion of the elongate power cable (32);
wherein the magnetic attraction between the at least one first magnetic portion and the at least one second magnetic portion (60, 72) draws the first and second coupler portions (50, 63) to one another and causes the first and second coupler portions (50, 63) to releasably couple and self align to one another when the first and second coupler portions (50, 63) are brought into close proximity to one another, such that the first electrical contact (58) makes electrical connection with the second electrical contact (70), thereby coupling the distal end (44) of the elongate power cable (32) to the proximal end (26) of the treatment apparatus (21).

2. The system of Claim 1, wherein the at least one first magnetic portion (60) and the at least one second magnetic portion (72) are selected from the group comprising a magnet and a ferromagnetic material.

3. The system of Claim 1 or Claim 2, further comprising a first aligner (62) located on the first coupler portion (50) and a second aligner (74) located on the second coupler portion (63), wherein the first and second aligner (62, 74) mate to maintain alignment between the first coupler portion (50) and the second coupler portion (63).

4. The system of Claim 3, wherein the first aligner (62) and the second aligner (74) are selected from the group comprising a protrusion and a recess.

5. The system of Claim 1, wherein the distal end (84) of the sleeve (80) is secured to at least one of the treatment apparatus (21) or the second coupler portion (63).

6. The system of any preceding claim, wherein the sleeve comprises a rigid or semi-rigid portion and a flexible portion.

7. The system of any preceding Claim, wherein the at least one first magnetic portion (60) is selected from a group comprising a pair of first magnets, a pair of first ferromagnetic materials, and a combination of a first magnet and a first ferromagnetic material.

8. The system of Claim 7, wherein the pair of first magnets, first ferromagnetic materials, or magnet and ferromagnetic material, are located adjacent diagonally opposite corners of a coupling area of the first coupler portion (50), and a mating pair of second magnets, second ferromagnetic material, or second magnet and second ferromagnetic material, are located adjacent diagonally opposite corners of a coupling area of the second coupler portion (63).

## Patentansprüche

1. System (20) zur elektrischen Kopplung, umfassend:
eine Behandlungsvorrichtung (21), die ein proximales Ende (26) und ein distales Ende (24) aufweist;
ein längliches Netzkabel (32), das ein proximales Ende (38), das zur Kopplung an eine Stromquelle (34) ausgelegt ist, und ein distales Ende (44) aufweist;
einen ersten Kopplerabschnitt (50) an dem distalen Ende (44) des Netzkabels (32), wobei der erste Kopplerabschnitt (50) zumindest einen ersten elektrischen Kontakt (58) und zumindest einen ersten magnetischen Abschnitt (60) aufweist;
einen zweiten Kopplerabschnitt (63) an dem proximalen Ende (26) der Behandlungsvorrichtung (21), wobei der zweite Kopplerabschnitt (63) zumindest einen zweiten elektrischen Kontakt (70) und zumindest einen zweiten magnetischen Abschnitt (72) aufweist, wobei der erste und zweite magnetische Abschnitt (60, 72) gegenseitig magnetisch anziehbar sind; und
eine Hülse (80), die ein proximales Ende (82) und ein distales Ende (84) aufweist, wobei die Hülse (80) ausgelegt ist, um zumindest einen Abschnitt des ersten und zweiten Kopplerabschnitts (50, 63) zu bedecken, wenn der erste und zweite Kopplerabschnitt (50, 63) aneinander gekoppelt sind, wobei die Hülse (80) einen eingefahrenen Zustand umfasst, in dem die Hülse (80) nur Abschnitte von zumindest einem von der Behandlungsvorrichtung (21) und dem zweiten Kopplerabschnitt (63) bedeckt, und einen erweiterten Zustand, in dem die Hülse (80) den ersten und zweiten Kopplerabschnitt (50, 63) und zumindest einen Abschnitt des länglichen Netzkabels (32) bedeckt;
wobei die magnetische Anziehung zwischen dem zumindest einen ersten magnetischen Abschnitt und dem zumindest einen zweiten magnetischen Abschnitt (60, 72) den ersten und zweiten Kopplerabschnitt (50, 63) gegenseitig anzieht und bewirkt, dass sich der erste und zweite Kopplerabschnitt (50, 63) lösbar aneinander koppeln und selbst ausrichten, wenn der erste und zweite Kopplerabschnitt (50, 63) in enge Nähe zueinander gebracht werden, sodass der erste elektrische Kontakt (58) eine elektrische Verbindung mit dem zweiten elektrischen Kontakt (70) herstellt, wodurch das distale Ende (44) des länglichen Netzkabels (32) an das proximale Ende (26) der Behandlungsvorrichtung (21) gekoppelt wird.

2. System nach Anspruch 1, wobei der zumindest eine erste magnetische Abschnitt (60) und der zumindest eine zweite magnetische Abschnitt (72) aus der Gruppe ausgewählt sind, die einen Magneten und ein ferromagnetisches Material umfasst.

3. System nach Anspruch 1 oder Anspruch 2, ferner umfassend einen ersten Ausrichter (62), der sich auf dem ersten Kopplerabschnitt (50) befindet, und einen zweiten Ausrichter (74), der sich auf dem zweiten Kopplerabschnitt (63) befindet, wobei der erste und zweite Ausrichter (62, 74) zusammenpassen, um die Ausrichtung zwischen dem ersten Kopplerabschnitt (50) und dem zweiten Kopplerabschnitt (63) beizubehalten.

4. System nach Anspruch 3, wobei der erste Ausrichter (62) und der zweite Ausrichter (74) aus der Gruppe ausgewählt sind, die einen Vorsprung und eine Aussparung umfasst.

5. System nach Anspruch 1, wobei das distale Ende (84) der Hülse (80) an zumindest einem von der Behandlungsvorrichtung (21) oder dem zweiten Kopplerabschnitt (63) befestigt ist.

6. System nach einem vorhergehenden Anspruch, wobei die Hülse einen festen oder halbfesten Abschnitt und einen flexiblen Abschnitt umfasst.

7. System nach einem vorhergehenden Anspruch, wobei der zumindest eine erste magnetische Abschnitt (60) aus einer Gruppe ausgewählt ist, die ein Paar erster Magneten, ein Paar erster ferromagnetischer Materialien und eine Kombination aus einem ersten Magneten und einem ersten ferromagnetischen Material umfasst.

8. System nach Anspruch 7, wobei sich das Paar erster Magneten, erster ferromagnetischer Materialien oder der Magnet und das ferromagnetische Material neben diagonal gegenüberliegenden Ecken eines Kopplungsbereichs des ersten Kopplerabschnitts (50) befinden und sich ein passendes Paar zweiter Magneten, zweiten ferromagnetischen Materials oder ein zweiter Magnet und ein zweites ferromagnetisches Material neben diagonal gegenüberliegenden Ecken eines Kopplungsbereichs des zweiten Kopplerabschnitts (63) befinden.

## Revendications

1. Système (20) pour le couplage électrique, comprenant :
un appareil de traitement (21) ayant une extrémité proximale (26) et une extrémité distale (24) ;
un câble d'alimentation allongé (32) ayant une extrémité proximale (38) configurée pour être couplée à une source d'énergie (34), et une extrémité distale (44) ;
une première partie de coupleur (50) au niveau de l'extrémité distale (44) du câble d'alimentation (32), la première partie de coupleur (50) ayant au moins un premier contact électrique (58) et au moins une première partie magnétique (60) ;
une seconde partie de coupleur (63) au niveau de l'extrémité proximale (26) de l'appareil de traitement (21), la seconde partie de coupleur (63) ayant au moins un second contact électrique (70) et au moins une seconde partie magnétique (72), dans lequel les première et seconde parties magnétiques (60, 72) peuvent être attirées magnétiquement l'une par rapport à l'autre ; et
un manchon (80) ayant une extrémité proximale (82) et une extrémité distale (84), le manchon (80) étant configuré pour recouvrir au moins une partie des première et seconde parties de coupleur (50, 63) lorsque la première et la seconde des parties de coupleur (50, 63) sont couplées l'une à l'autre, dans lequel le manchon (80) comprend un état rétracté dans lequel le manchon (80) ne recouvre que des parties d'au moins un des appareils de traitement (21) et la seconde partie de coupleur (63), et un état étendu dans lequel le manchon (80) recouvre les première et seconde parties de coupleur (50, 63) et au moins une partie du câble d'alimentation allongé (32) ;
dans lequel l'attraction magnétique entre l'au moins une première partie magnétique et l'au moins une seconde partie magnétique (60, 72) attire les première et seconde parties de coupleur (50, 63) les unes par rapport aux autres et amène les première et seconde parties de coupleur (50, 63) à se coupler et à s'aligner de manière amovible l'un sur l'autre lorsque les première et seconde parties de coupleur (50, 63) sont rapprochées les unes des autres, de sorte que le premier contact électrique (58) établit une connexion électrique avec le second contact électrique (70), couplant ainsi l'extrémité distale (44) du câble d'alimentation allongé (32) à l'extrémité proximale (26) de l'appareil de traitement (21).

2. Système selon la revendication 1, dans lequel l'au moins une première partie magnétique (60) et l'au moins une seconde partie magnétique (72) sont choisies dans le groupe comprenant un aimant et un matériau ferromagnétique.

3. Système selon la revendication 1 ou la revendication 2, comprenant en outre un premier aligneur (62) situé sur la première partie de coupleur (50) et un second aligneur (74) situé sur la seconde partie de coupleur (63), dans lequel les premier et second aligneurs (62, 74) s'accouplent pour maintenir l'alignement entre la première partie de coupleur (50) et la seconde partie de coupleur (63).

4. Système selon la revendication 3, dans lequel le premier aligneur (62) et le second aligneur (74) sont choisis dans le groupe comprenant une protubérance et un évidement.

5. Système selon la revendication 1, dans lequel l'extrémité distale (84) du manchon (80) est fixée à au moins l'un de l'appareil de traitement (21) ou de la seconde partie de coupleur (63).

6. Système selon l'une quelconque des revendications précédentes, dans lequel le manchon comprend une partie rigide ou semi-rigide et une partie flexible.

7. Système selon l'une quelconque des revendications précédentes, dans lequel l'au moins une première partie magnétique (60) est choisie parmi un groupe comprenant une paire de premiers aimants, une paire de premiers matériaux ferromagnétiques et une combinaison d'un premier aimant et d'un premier matériau ferromagnétique.

8. Système selon la revendication 7, dans lequel la paire de premiers aimants, de premiers matériaux ferromagnétiques, ou d'aimant et de matériau ferromagnétique, sont situés adjacents aux coins diagonalement opposés d'une zone de couplage de la première partie de coupleur (50), et une paire de couplage de seconds aimants, d'un second matériau ferromagnétique, ou d'un deuxième aimant et un second matériau ferromagnétique, sont situés adjacents aux coins diagonalement opposés d'une zone de couplage de la seconde partie de coupleur (63).
